(19) Europäisches Patentamt
European Patent Office
Office européen des brevets

(11)  EP 2 579 025 B1

(12)  **EUROPEAN PATENT SPECIFICATION**

(45) Date of publication and mention
of the grant of the patent:
**28.09.2016  Bulletin 2016/39**

(51) Int Cl.:
$G01N\ 21/64$ (2006.01)        $G01N\ 33/543$ (2006.01)
$C03C\ 17/40$ (2006.01)

(21) Application number: **11792392.0**

(86) International application number:
**PCT/JP2011/062919**

(22) Date of filing: **06.06.2011**

(87) International publication number:
**WO 2011/155435 (15.12.2011 Gazette 2011/50)**

(54) **NEAR FIELD-ENHANCED FLUORESCENCE SENSOR CHIP**

NAHFELDVERSTÄRKTER FLUORESZENZ-SENSORCHIP

PUCE DE DÉTECTION EN FLUORESCENCE AMPLIFIÉE DE CHAMP PROCHE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.06.2010  JP 2010129943**

(43) Date of publication of application:
**10.04.2013  Bulletin 2013/15**

(73) Proprietor: **Konica Minolta Holdings, Inc.
Tokyo 100-7015 (JP)**

(72) Inventors:
• **KAYA, Takatoshi**
**Hino-shi**
**Tokyo 191-8511 (JP)**
• **HIKAGE, Naoki**
**Hino-shi**
**Tokyo 191-8511 (JP)**
• **NINOMIYA, Hidetaka**
**Hino-shi**
**Tokyo 191-8511 (JP)**

(74) Representative: **Gille Hrabal
Brucknerstrasse 20
40593 Düsseldorf (DE)**

(56) References cited:
**EP-A1- 1 143 251        JP-A- 6 265 336
JP-A- H04 501 605        JP-A- 2003 247 935
JP-A- 2009 079 970        US-A1- 2006 110 594
US-A1- 2008 187 462**

• YU F ET AL: "SURFACE PLASMON
FLUORESCENCE IMMUNOASSAY OF FREE
PROSTATE-SPECIFIC ANTIGEN IN HUMAN
PLASMA AT THE FEMTOMOLAR LEVEL",
ANALYTICAL CHEMISTRY, AMERICAN
CHEMICAL SOCIETY, US, vol. 76, no. 22, 15
November 2004 (2004-11-15), pages 6765-6770,
XP001225811, ISSN: 0003-2700, DOI:
10.1021/AC048937W
• FANG YU ET AL.: 'Surface Plasmon Fluorescence
Immunoassay of Free Prostate-Specific Antigen
in Human Plasma at the Femtomolar Level' ANAL.
CHEM. vol. 76, no. 22, 2004, pages 6765 - 6770,
XP001225811

EP 2 579 025 B1

**Description**

Technical Field

The present invention relates to a surface plasmon-field enhanced fluorescence spectroscopy [SPFS] sensor chip, namely, a near field-enhanced fluorescence sensor chip.

Background Art

SPFS is a method capable of detecting an analyte in an extremely slight amount and/or an extremely low concentration by generating compression waves (surface plasmons) on a metal thin film surface that is in contact with a dielectric under the conditions that laser light used for irradiation undergoes attenuated total reflection [ATR] on the metal thin film surface, to increase the quantity of photons of the laser light several tens to several hundreds times (electrical field enhancement effect of surface plasmons) and thereby efficiently exciting a fluorescent dye in the vicinity of the metal thin film.
On the other hand, surface plasmon resonance [SPR] is a phenomenon that when the wave number of compression waves (surface plasmons) generated on a metal thin film surface that is in contact with a dielectric under the conditions that laser light used for irradiation undergoes ATR on the metal thin film surface and the wave number of evanescent waves which are liable to be influenced by a difference of dielectric constant (or refractive index) coincide with each other, they resonate with each other and the reflected light is attenuated. By utilizing SPR, a ligand and an analyte interact with each other on the sensor surface, whereby a difference in the dielectric constant (or refractive index) of the dielectric is made. As a result, the surface plasmon resonance varies, and thereby, the interaction between the ligand and the analyte can be quantitatively determined.
Sensor substrates for use in such SPFS and SPR are described in patent literatures 1 and 2, respectively.
In the patent literature 1, such a sensor unit (100) as shown in Fig. 7 is disclosed. The sensor unit is designed to comprise a transparent plate made of glass, plastic or another transparent material, a metal film formed on one surface of the plate by sputtering, a dextran layer (dextran film) bonded to the metal film, and a ligand bonded to the dextran film. This ligand undergoes interaction with a specific biomolecule (e.g., antigen) present in a sample solution, and variable angle internal total reflection fluorescence emission, namely, SPFS, can be carried out.
In the patent literature 2, technique of using such a measuring chip (200) as shown in Fig. 8 and a bioactive substance bonded to the surface of the measuring chip by a covalent bond in SPR is described. The measuring chip comprises a dielectric block, a metal film formed on one surface of the dielectric block, a hydrophobic polymer compound for coating the metal film, and a hydrogel for coating the surface of the polymer compound.
In the patent literatures 1 and 2, however, there is neither description nor suggestion about the ligand and the density of the dextran or the like for immobilizing the ligand, and it was found that SPFS sensor chips for SPFS prepared based on the descriptions of the patent literatures 1 and 2 show variation in a signal depending upon the chip used for the assay.
In US 2008/0187462 A1 substrates are described which have, on the surface thereof, a physiologically active substance that has been immobilized thereon via a hydrophilic polymer layer formed with hydrophilic polymers. Preferably, the substrates have a metal film. Preferably, the substrates are used in surface plasmon resonance analysis. In one embodiment fluorescently-labeling substances that have been known to bind to physiologically active substance are immobilized on a sensor substrate, and then fluorescence intensity is measured using a fluorescence microscope or the like.

Citation List

Patent Literature

Patent literature 1: Japanese Patent No. 3,294, 605 (WO90/05295
Patent literature 2: Japanese Patent No. 4,292,043(JPA2005-987770)

Summary of Invention

Technical Problem

In order to solve, for example, the problem that even if the immobilization density of the ligand (antibody) contained in the sensor unit described in the patent literature 1 is increased, there is a large variability of signal intensity of each chip relative to that the assay signal is not increased so much, the object of the present invention is to provide an SPFS sensor chip capable of maximizing the efficiency of an antigen-antibody reaction or detection of fluorescence, an assay method using the sensor chip, an assay device and an assay kit.

Solution to Problem

From the analysis by the present inventors, it has been found that the signal intensity and its variability are influenced in SPFS measurement by the density of a ligand such as antibody, the material of a solid phase layer for immobilizing the ligand (type of a polymer such as dextran), the film thickness of the solid phase layer, and the film thickness of a structure consisting of the solid phase layer and the ligand, which contains the ligand on the solid phase layer. The present inventors have found that the signal of an assay is stabilized and increased by setting a fluctuation ratio of a half-width which varies depending upon the above conditions and is obtained from a graph on which the quantity of reflected light measured by a light quantity detector is plotted against the light source incident angle, in a specific numerical range, and they have accomplished the present invention.

That is to say, the SPFS sensor chip according to the present invention, as defined in appended claim 1, is a surface plasmon-field enhanced fluorescence spectroscopy [SPFS], sensor chip comprising a transparent support, a metal thin film formed on one surface of the transparent support, a self-assembled monolayer [SAM] formed on a surface of the metal thin film, said surface not being in contact with the transparent support, a solid phase layer formed on a surface of the SAM and having a three-dimensional structure, said surface not being in contact with the metal thin film, wherein the solid phase layer contains a hydrophilic polymer, and a ligand immobilized in the solid phase layer, characterized in that the SPFS sensor chip has a fluctuation ratio, represented by the following formula , in the range from 0 to 30%,

$$\{\text{half-width } (\alpha) - \text{half-width } (\beta)\}/\text{half-width } (\beta) \times 100$$

wherein the half-width ($\alpha$) is a half-width obtained from a graph on which the quantity of reflected light of that light entering one surface of the transparent support at a prescribed angle, said surface not being in contact with the metal thin film, as measured by a light quantity detector (e. g. , photodiode [PD]) placed on the other surface side of the transparent support, is plotted against the angle, and the half-width ($\beta$) is a half-width of a substrate, the substrate includes the transparent support and the metal thin film formed on one surface of the transparent support, where the transparent support is not together with the SAM, the solid phase layer and the ligand of the SPFS sensor chip.

The solid phase layer preferably contains glucose, carboxymethylated glucose and a polymer constituted of at least one monomer selected from a group consisting of monomers included in vinyl esters, acrylic acid esters, methacrylic acid esters, olefins, styrenes, crotonic acid esters, itaconic acid diesters, maleic acid diesters, fumaric acid diesters, allyl compounds, vinyl ethers and vinyl ketones, respectively.

According to the invention, the solid phase layer has a density of less than 2 ng/mm$^2$.

According to the invention, the solid phase layer has a mean film thickness of 3 to 80 nm.

According to the invention, the density of the ligand immobilized in the solid phase layer is in the range from 10 femto-mol/cm$^2$ to 100 pico-mol/cm$^2$.

An exemplary assay method comprises at least the following steps (a) to (d):

step (a) : a step of bringing a specimen into contact with the SPFS sensor chip according to the present invention,
step (b) : a step of further allowing a conjugate of a ligand which may be the same as or different from the ligand contained in the SPFS sensor chip and a fluorescent dye to react with the SPFS sensor chip having passed the step (a),
step (c): a step of irradiating the SPFS sensor chip having passed the step (b) with laser light from the other surface of the transparent support, on said surface the metal thin film not being formed, to measure the quantity of fluorescence emitted from the excited fluorescent dye, and
step (d): a step of calculating the quantity of an analyte contained in the specimen from the measurement result obtained in the step (c).

In the exemplary assay method, the conjugate is preferably bonded to the analyte.

The analyte may be a tumor marker or carcinoembryonic antigen.

An exemplary assay device uses the SPFS sensor chip and is used in the assay method.

An exemplary assay kit comprises at least a sensor chip substrate which comprises a transparent support, a metal thin film formed on one surface of the transparent support, SAM formed on a surface of the metal thin film, said surface not being in contact with the transparent support, and a solid phase layer formed on a surface of the SAM and having a three-dimensional structure, said surface not being in contact with the metal thin film, and which is used in the SPFS sensor chip.

Advantageous Effects of Invention

When the SPFS sensor chip according to the present invention , as defined in appended claim 1, is used for an assay, the signal is free from non-uniformity (that is, coefficient of variation (CV) is extremely small and is stable), and the signal is increased, in spite of that the SPFS sensor chip uses a solid phase layer containing dextran or the like, and hence, improvement in detection stability and high-sensitive detection can be achieved.

Brief Description of Drawings

[Fig. 1] Fig. 1 is a longitudinal sectional view schematically showing a preferred embodiment of the SPFS sensor chip according to the present invention.

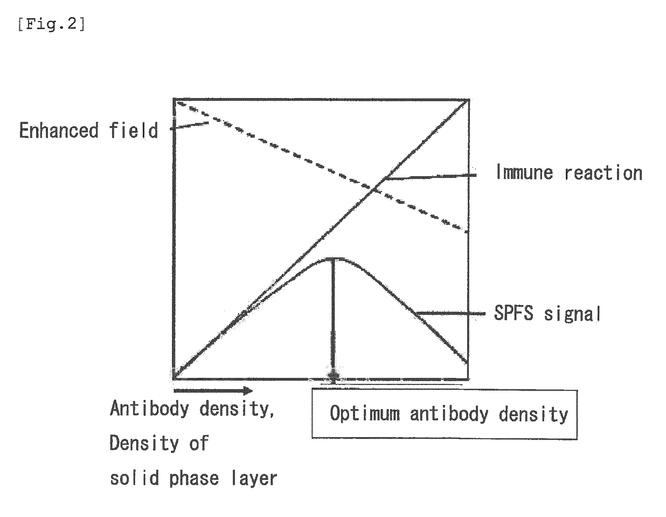

[Fig. 2] Fig. 2 shows ideal graphs on which the density of a solid phase layer (i.e., density of antibody immobilized in the solid phase layer) obtained in the SPFS measurement using the SPFS sensor chip shown in Fig. 1 is plotted as abscissa and the enhanced field, the immune reaction and the SPFS signal obtained in the same SPFS measurement are plotted as ordinate.
[Fig. 3] Figs. 3(A) to 3(C) schematically show longitudinal sectional views of SPFS sensor chips produced in Comparative Example 1, Comparative Example 2 and Example 1, respectively.

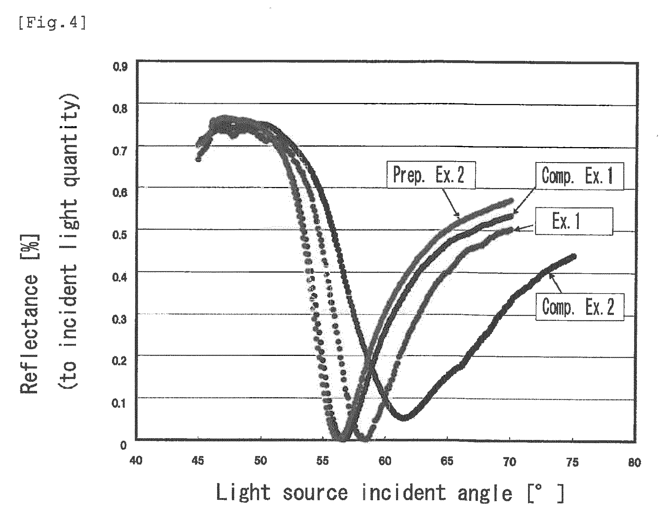

[Fig. 4] Fig. 4 shows graphs on which the reflectance (to the quantity of incident light) measured by a photodiode at a light source incident angle is plotted against the light source incident angle in the case of using SPFS sensor chips and a substrate produced in Example 1, Comparative Examples 1 and 2, and Preparation Example 2, respectively.

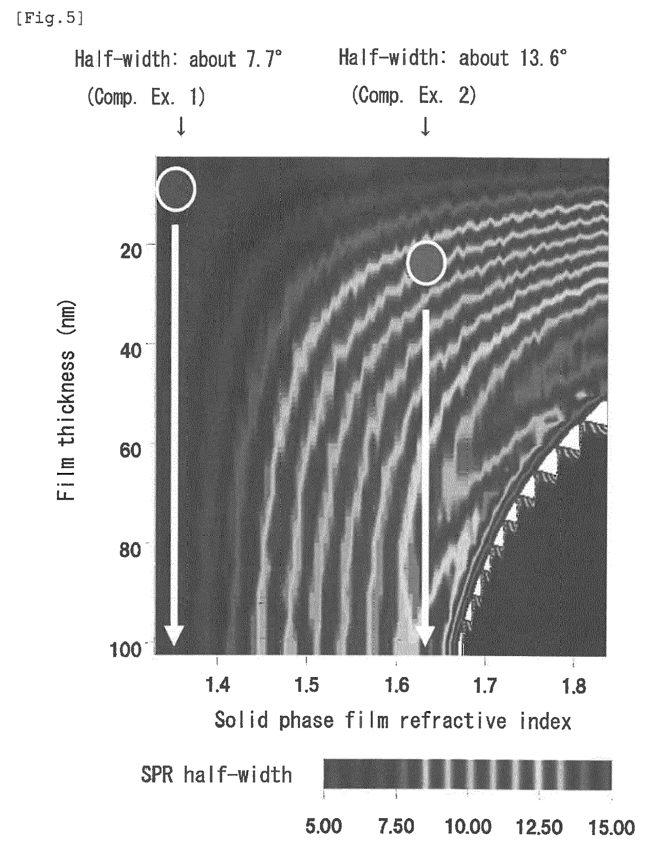

[Fig. 5] Fig. 5 shows graphs on which the film thickness measured by an atomic force microscope [AFM] is plotted against the refractive index of the solid phase layer in the case of using SPFS sensor chips produced in Comparative Examples 1 and 2, respectively.
[Fig. 6] Fig. 6 shows a graph on which "Signal" [a.u.] is plotted against the density [mol/cm$^2$] of an antibody that is a ligand immobilized in the solid phase layer in one embodiment of the SPFS sensor chip according to the present invention.

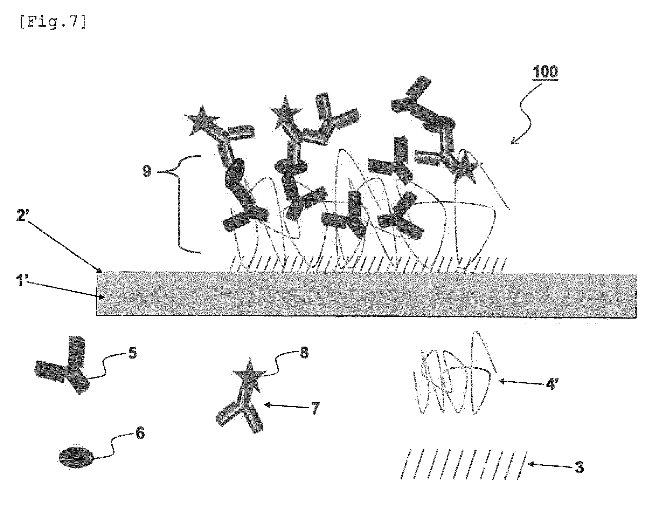

[Fig. 7] Fig. 7 schematically shows a longitudinal sectional view of a sensor unit disclosed in the patent literature 1.

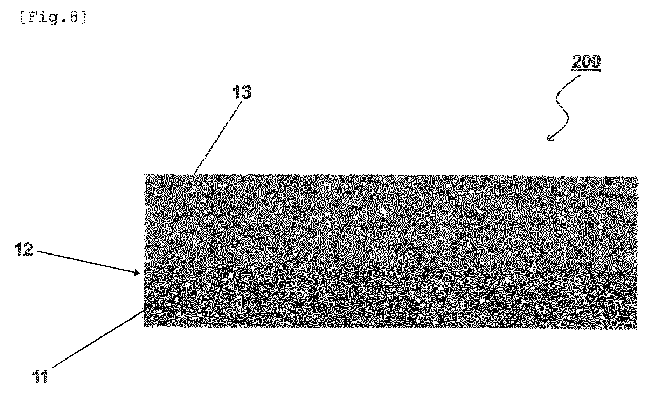

[Fig. 8] Fig. 8 schematically shows a longitudinal sectional view of a measuring chip disclosed in the patent literature 2. Description of Embodiments

A SPFS sensor chip according to the present invention, as defined by appended claim 1, and the process for producing the same are described in detail hereinafter.

<SPFS sensor chip>

The SPFS sensor chip according to the present invention , as defined by appended claim 1, is an "SPFS sensor chip" comprising a transparent support, a metal thin film formed on one surface of the transparent support, SAM formed on a surface of the thin film, said surface not being in contact with the transparent support, a solid phase layer formed on a surface of the SAM and having a three-dimensional structure, said surface not being in contact with the thin film, wherein the solid phase layer contains a hydrophilic polymer, and a ligand immobilized inside the solid phase layer and onto the outer surface thereof, characterized in that the SPFS sensor chip has a fluctuation ratio, represented by the following formula , in the range from 0 to 30%,

$$\{\text{half-width } (\alpha) - \text{half-width } (\beta)\}/\text{half-width } (\beta) \times 100$$

wherein (i) the solid phase layer has a density of less than 2 ng/mm$^2$ ; (ii) the solid phase layer has a mean film thickness of 3 to 80 nm;(iii) the density of the ligand immobilized in the solid phase layer is in the range from 10 femto-mol/cm$^2$ to 100 pico-mol/cm$^2$ ; (iv) the half-width ($\alpha$) is a half-width obtained from a graph on which the quantity of reflected light of that light entering one surface of the transparent support at a prescribed angle, said surface not being in contact with the metal thin film, as measured by a light quantity detector (e.g., photodiode [PD]) placed on the other surface side of the transparent support, is plotted against the angle, and the half-width ($\beta$) is a half-width of a substrate, the substrate includes the transparent support and the metal thin film formed on one surface of the transparent support.

That is to say, when the half-width of the SPFS sensor chip according to the present invention is taken as a "half-width ($\alpha$)" and the half-width of a substrate excluding the SAM, the solid phase layer and the ligand immobilized in the solid phase layer from the SPFS sensor chip according to the present invention, namely, a half-width of a substrate consisting of the transparent support and the metal thin layer, is taken as a "half-width ($\beta$)", the fluctuation ratio defined by {half-width ($\alpha$) - half-width ($\beta$)}/half-width ($\beta$) $\times$ 100 is not less than 0% but not more than 30%, preferably not less than 0% but not more than 20%, more preferably not less than 0.001% but not more than 20%. In general, thehalf-width ($\alpha$) and the half-width ($\beta$) satisfy the condition of half-width ($\alpha$)>half-width ($\beta$).

The "half-width" used in the present inventionmeans a half-width defined in the followingmanner. That is to say, in the above-mentioned graph, when the quantity of reflected light [PD output (V)] that is on the y axis is plotted against the light source incident angle (°) that is on the x axis in an orthogonal plane coordinate, a downward convex curve is drawn, and the half-width means a width of the convex curve in the direction of the x axis at a value obtained by dividing the sum of a maximum value and a minimum value of the quantity of reflected light by 2 (i.e., half value).

If the fluctuation ratio exceeds the upper limit of the above numerical range, the degree of field enhancement is lowered and there is a fear of lowering of the SPFS signal when the SPFS sensor chip is used for an assay method. As this fluctuation ratio approaches 0%, the SPFS sensor chip comes to attain the effect of the present invention with a higher level, and an SPFS sensor chip having a fluctuation ratio of 0% can be said to be an ideal one.

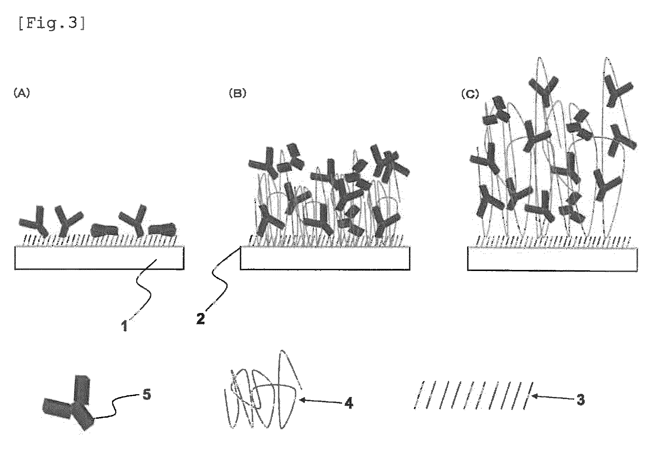

In the present specification, the substrate consisting of the transparent support and the metal thin film that are laminated in this order is referred to as a "substrate" simply, and the substrate consisting of the transparent support, the metal thin film and the SAM that are laminated in this order is particularly referred to as a "sensor substrate", and a structure in which the solid phase layer has been formed on the SAM surface of the sensor substrate is particularly referred to as a "sensor chip substrate". A structure in which a ligand has been immobilized onto the SAM of the "sensor substrate" without laminating a solid phase layer on the SAM is also referred to as a "SPFS sensor chip" here, and such a sensor chip is, for example, a SPFS sensor chip of Fig. 3(A), but this is different from the "SPFS sensor chip" according to the present invention. A structure in which a ligand has been immobilized in the solid phase layer of the "sensor chip substrate" corresponds to the "SPFS sensor chip" according to the present invention.

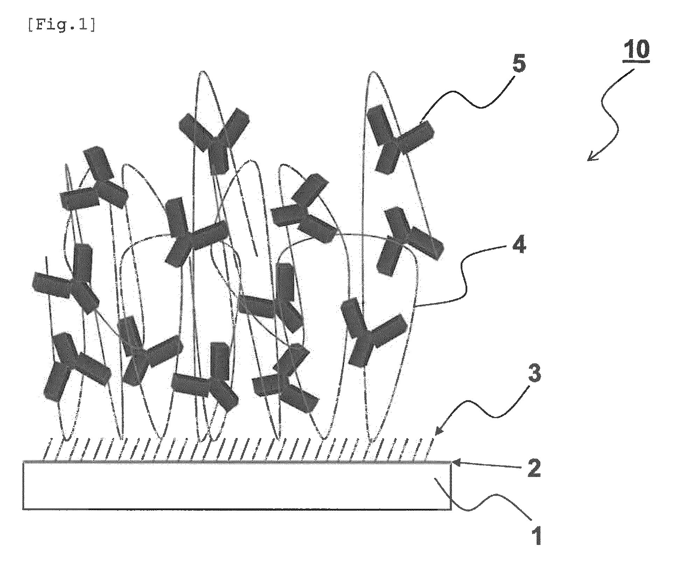

Preferred embodiments of the SPFS sensor chips according to the present invention are shown in Fig. 1 and Fig. 3C. As shown in Fig. 1 and Fig. 3(C), individual carboxymethyl dextran [CMD] for forming the solid phase layer having a three-dimensional structure may be immobilized onto the sensor substrate in a nearly vertical state, or one dextran may be immobilized onto the sensor substrate at plural positions, and the present invention is not limited to these embodiments.

(Transparent support)

In the present invention, as a support for supporting the structure of the SPFS sensor chip, a transparent support is used. The reason why the transparent support is used as the support in the present invention is that light irradiation of the later-described metal thin film is carried out through this transparent support.

There is no specific limitation on the material of the transparent support for use in the present invention as far as the object of the present invention is achieved. For example, this transparent support may be made of glass or may be made of plastic such as polycarbonate [PC] or a cycloolefin polymer [COP].

The refractive index [$n_d$] of the transparent support at the d line (588 nm) is preferably 1.40 to 2.20, and the thickness thereof is preferably 0.01 to 10 mm, more preferably 0.5 to 5 mm. The size (length $\times$ width) of the transparent support is not specifically restricted.

As commercial products of the glass transparent supports, "BK7" (refractive index [$n_d$] : 1.52) and "LaSFN9" (refractive index [$n_d$] : 1.85) manufactured by Schott Japan Corporation, "K-PSFn3" (refractive index [$n_d$] : 1.84), "K-LsSFn17" (refractive index [$n_d$] : 1.88) and "K-LaSFn22" (refractive index [$n_d$] : 1.90) manufactured by Sumita Optical Glass, Inc., and "S-LAL10" (refractive index [$n_d$] : 1.72) manufactured by Ohara Inc. or the like are preferable from the viewpoints of optical properties and cleanability.

The surface of the transparent support is preferably subjected to cleaning with acid and/or plasma before the metal thin film is formed on the surface.

The cleaning treatment with acid is preferably carried out by immersing the transparent support in 0.001 to 1 N hydrochloric acid for 1 to 3 hours.

As the cleaning treatment with plasma, there can be mentioned, for example, a method of immersing the transparent support in a plasma dry cleaner ("PDC200" manufactured by Yamato Scientific Co., Ltd.) for 0.1 to 30 minutes.

(Metal thin film)

In the SPFS sensor chip according to the present invention, a metal thin film is formed on one surface of the transparent support. This metal thin film plays a role in causing surface plasmon excitation due to the irradiation with light from a light source, generating electric field and bringing about light emission from a fluorescent dye.

The metal thin film formed on one surface of the transparent support is preferably made of at least one metal selected from the group consisting of gold, silver, aluminum, copper and platinum, and is more preferably made of gold. These metals may be in the form of their alloys. Such metal species are preferable because they are stable to oxidation and field enhancement by the surface plasmons is promoted.

When a glass support is used as the transparent support, it is preferable to form a thin film of chromium, a nickel-chromium alloy or titanium in advance in order to more firmly bond the metal thin film to the glass.

Examples of methods to form the metal thin film on the transparent support include sputtering method, deposition method (deposition by resistance heating, deposition by electron rays, etc.), electroplating method and electroless plating method. From the viewpoint of easy control of thin film-forming conditions, it is preferable to form a thin film of chromium and/or a metal thin film by sputtering or deposition method.

The thickness of the metal thin film is preferably as follows; gold: 5 to 500 nm, silver: 5 to 500 nm, aluminum: 5 to 500 nm, copper: 5 to 500 nm, platinum: 5 to 500 nm, and their alloys: 5 to 500 nm. The thickness of the thin film of chromium is preferably 1 to 20 nm.

From the viewpoint of field enhancement effect, the thickness of the metal thin film is more preferably as follows; gold: 20 to 70 nm, silver: 20 to 70 nm, aluminum: 10 to 50 nm, copper: 20 to 70 nm, platinum: 20 to 70 nm, and their alloys: 10 to 70 nm. The thickness of the thin film of chromium is more preferably 1 to 3 nm.

When the thickness of the metal thin film is in the above range, surface plasmons are easily generated, so that such a thickness is preferable. The size (length × width) of the metal thin film is not specifically restricted.

(SAM)

SAM [self-assembled monolayer] is formed on a surface of the metal thin film, said surface not being in contact with the transparent support, as a scaffold for immobilizing the solid phase layer, or for the purpose of preventing quenching of molecular fluorescence due to the metal in the case where the SPFS sensor chip is used for an assay method.

As the monomolecules to constitute the SAM, carboxyalkanethiol of about 4 to 20 carbon atoms (available from, for example, Dojindo Laboratories or Sigma-Aldrich Japan Inc.) is usually used, and 10-carboxy-1-decanethiol is particularly preferably used. The carboxyalkanethiol of 4 to 20 carbon atoms is preferable because SAM formed by the use of it is rarely influenced optically, that is, the SAM has properties of high transparency, low refractive index and small film thickness.

The method for forming such SAM is not specifically restricted, and hitherto publicly known methods are employable. For example, a method of immersing the transparent support having the metal thin film formed on its surface in an ethanol solution containing 10-carboxy-1-decanethiol (manufactured by Dojindo Laboratories) can be mentioned. The thiol group of the 1-carboxy-1-decanethiol is bonded to the metal, immobilized and self-assembled on the surface of the metal thin film to form SAM.

Prior to formation of the SAM, a "spacer layer composed of a dielectric" may be formed, and in this case, as the monomolecules to constitute the SAM, a silane coupling agent having an ethoxy group (or methoxy group) that is hydrolyzed to form a silanol group [Si-OH] and having, at the other end, a reactive group such as amino group, glycidyl group or carboxyl group is preferably used.

The method for forming such SAM is not specifically restricted, and hitherto publicly known methods are employable.

As the dielectrics for use in the formation of such a "spacer layer composed of a dielectric", optically transparent various inorganic substances, and natural or synthetic polymers can be also used. Of these, silicon dioxide [$SiO_2$], titanium dioxide [$TiO_2$] or aluminum oxide [$Al_2O_3$] is preferably contained because they are excellent in chemical stability, production stability and optical transparency.

The thickness of the spacer layer composed of a dielectric is usually 10 nm to 1 mm, and from the viewpoint of stability of resonance angle, the thickness is preferably not more than 30 nm, more preferably 10 to 20 nm. On the other hand, from the viewpoint of field enhancement, the thickness is preferably 200 nm to 1 mm, and from the viewpoint of stability of field enhancement effect, the thickness is more preferably 400 nm to 1,600 nm.

Examples of methods for forming the spacer layer composed of a dielectric include sputtering method, deposition method by electron rays, thermal deposition method, method by chemical reaction using a material such as polysilazane, and coating method using spin coater.

(Solid phase layer)

The solid phase layer is formed on a surface of the SAM, said surface not being in contact with the metal thin film, and has a three-dimensional structure.

This "three-dimensional structure" refers to a structure of a solid phase layer in which immobilization of the later-described ligand is not limited to two dimensions of the surface (and its vicinity) of the "sensor substrate" and is extended to the three-dimensional space separated from the substrate surface.

Such a solid phase layer according to the invention contains a hydrophilic polymer, and preferably contains glucose, carboxymethylated glucose and a polymer constituted of at least one monomer selected from the group consisting of monomers included in vinyl esters, acrylic acid esters, methacrylic acid esters, olefins, styrenes, crotonic acid esters, itaconic acid diesters, maleic acid diesters, fumaric acid diesters, allyl compounds, vinyl ethers and vinyl ketones, respectively; it more preferably contains a hydrophilic polymer, such as dextran or a dextran derivative, and a hydrophobic polymer constituted of a hydrophobic monomer included in vinyl esters, acrylic acid esters, methacrylic acid esters, olefins, styrenes, crotonic acid esters, itaconic acid diesters, maleic acid diesters, fumaric aciddiesters, allyl compounds, vinyl ethers and vinyl ketones, respectively; and dextran such as carboxymethyl dextran [CMD] is particularly preferable from the viewpoints of biocompatibility, inhibition of non-specific adsorption reaction and high hydrophilicity.

The molecular weight of CMD is preferably not less than 1 kDa but not more than 5, 000 kDa, more preferably not less than 4kDa but not more than 1.000 kDa.

The solid phase layer (composed of, for example, dextran or a dextran derivative) has a density of less than 2 $ng/mm^2$. The density of the solid phase layer can be properly controlled according to the type of the polymer used. It is preferable that the polymer is immobilized on the SAM described above in such density range, because an assay signal is stabilized and increased in the case where the SPFS sensor chip is used for an assay method. The density of "Sensor Chip CM5" manufactured by Biacore Life Sciences was 2 $ng/mm^2$. This density was determined in the following manner. Using this CM5 substrate and a substrate with only a gold film, a mean 2000 RU was measured at a measurement signal obtained by the use of SPR measuring equipment manufactured by Biacore Life Sciences, and as a result, the density was estimated to be 2 $ng/mm^2$.

The mean film thickness of the solid phase layer is in the range from 3 to 80 nm. This film thickness can be measured by an atomic force microscope [AFM] or the like. According to the invention the mean of the film thickness of the solid phase layer is in such a range, because an assay signal is stabilized and increased in the case where the SPFS sensor chip is used for an assay method.

The method for forming the solid phase layer is described in detail in the later-described section of "Process for producing SPFS sensor chip>.

(Ligand)

In the present invention, the ligand is immobilized in the solid phase layer, and preferably inside the solid phase layer and onto the outer surface thereof, that is, the ligand is dispersed and immobilized in the three-dimensional structure of the solid phase layer, and when the SPFS sensor chip according to the present invention is used in the exemplary assay method, the ligand is used for the purpose of fixing (capturing) an analyte contained in the specimen.

In order to distinguish the ligand from a ligand of a "conjugate of a ligand and a fluorescent dye" used in the exemplary assay method, the ligand used in the SPFS sensor chip according to the present invention is referred to as a "first ligand" hereinafter, and the ligand used in the exemplary assay method is referred to as a "second ligand" hereinafter in the present specification. The first ligand and the second ligand may be the same as or different from each other.

In an exemplary embodiment of the present invention, the first ligand is a molecule or a molecular fragment capable of specifically recognizing (or being recognized by) the analyte contained in the specimen and being bonded thereto. Examples of such "molecules" or "molecular fragments" include nucleic acids (DNA, RNA, polynucleotide, oligonucleotide, PNA (peptide nucleic acid), etc. which may be single-stranded or double-stranded, or nucleoside, nucleotide and their modified molecules), proteins (polypeptide, oligopeptide, etc.), amino acids (including modified amino acids), saccharides (oligosaccharide, polysaccharides, sugar chain, etc.), lipid, and modified molecules and a complex of these substances, without limiting thereto.

The "protein" is, for example, an antibody, and examples thereof include anti-$\alpha$-fetoprotein [AFP] monoclonal antibody (available from Japan Clinical Laboratories, Inc., etc.), anti-carcinoembryonic antigen [CEA] monoclonal antibody, anti-CA19-9 monoclonal antibody and anti-PSA monoclonal antibody.

In the present invention, the term "antibody" includes polyclonal antibody or monoclonal antibody, antibody obtained by gene recombination, and antibody fragment.

Examples of methods to immobilize the first ligand include a method comprising converting a carboxyl group of a polymer having a reactive functional group, such as carboxymethyl dextran [CMD] to an active ester, by the use of water-soluble carbodiimide [WSC] (e. g., 1-ethyl-3-(3-dimethylaminopropyl)carbodiimide hydrochloride [EDC]) and N-hydroxysuccinic acid imide [NHS] and subjecting the carboxyl group that is coverted to active ester as described above and the amino group of the first ligand to dehydration reaction by the use of water-soluble carbodiimide to immobilize the ligand; and a method comprising subjecting the carboxyl group of the SAM and the amino group of the first ligand to dehydration reaction as described above to immobilize the ligand.

In order to prevent the later-described specimen or the like from being non-specifically adsorbed by the SPFS sensor chip, it is preferable to treat the surface of the SPFS sensor chip with a blocking agent such as bovine serum albumin [BSA] after the ligand is immobilized.

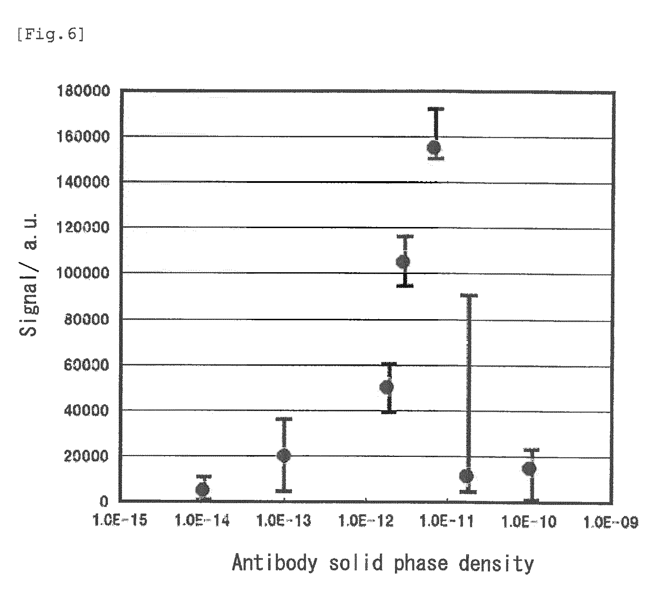

The density of the ligand having been immobilized inside the solid phase layer and onto the outer surface thereof is in the range from 1 femto-mol/cm$^2$ to 1 nano-mol/cm$^2$, more preferably in the range from 10 femto-mol/cm$^2$ to 100 pico-mol/cm$^2$. According to the present invention, the density of the ligand is in the above range, because the signal intensity is increased as shown in Fig. 6.

<Process for producing SPFS sensor chip>

The exemplary process for producing the SPFS sensor chip comprises at least a step of immersing a sensor substrate including a transparent support, a metal thin film formed on one surface of the support, and SAM formed on a surface of the thin film, said surface not being in contact with the support, in anMES-bufferedphysiological salt solution (ionic strength: not less than 0.1 mM but not more than 300 mM) which contains the aforesaid polymer having a molecular weight of not less than 1 kDa but not more than 5,000 kDa in an amount of not less than 0.01 mg/mL but not more than 100 mg/mL, N-hydroxysuccinic acid imide [NHS] in an amount of not less than 0.01 mM but not more than 300 mM and water-soluble carbodiimide [WSC] in an amount of not less than 0.01 mM but not more than 500 mM and has pH of not less than 4.0 but not more than 6.5, for not shorter than 0.2 hours but not longer than 3.0 hours.

From a huge number of experiments, the present inventors have found that when CMD is used as the polymer, the film thickness, the density, etc. of the CMD can be arbitrarily determined by controlling the molecular weight and the concentration of CMD and composition of a buffer solution (physiological salt solution) used for the reaction. It is also known that if the conditions to immobilize the CMD vary, the antibody solid phase density and the solid phase film thickness vary even though the subsequent conditions to immobilize the antibody are the same.

In other words, the present inventors have made trial and error regarding the combination of the antibody solid phase density and the solid phase film thickness, and they have grasped all the combinations of them by which the fluctuation ratio represented by the formula {half-width ($\alpha$) - half-width ($\beta$)}/half-width ($\beta$) $\times$ 100 satisfies 0 to 30%.

In the SPFS measurement, the non-uniform local electric field present at a height of several hundreds nm in the extreme vicinity of the sensor surface, the immune reaction attributed to the solid phase antibody, and the phenomenon attributed to the antibody solid phase density, such as lowering of enhanced field, participate in one another complicatedly, and the final fluorescence signal is output. On that account, it appears that the signal intensity correlation is not determined unconditionally by each parameter such as film thickness or antibody solid phase density, and they complicatedly participate in one another.

That is to say, this step is a step to obtain a "sensor chip substrate" by forming a solid phase layer having a three-dimensional structure on the other surface of the SAM, said surface not being in contact with the metal thin film.

The step to produce the "sensor substrate" in which a transparent support, a metal thin film and SAM are laminated in this order and the step to obtain the SPFS sensor chip according to the present invention by immobilizing the ligand onto the sensor chip substrate are as described previously.

The sensor chip substrate production step using carboxymethyl dextran [CMD] as the polymer contained in the solid phase layer is described below in detail. The polymer used in this step of the production process according to the present invention is not limited to CMD.

That is to say, the "sensor substrate" in which a transparent support, a metal thin film and SAM are laminated in this order is immersed in an MES-buffered physiological salt solution [MES] containing such carboxymethyl dextran as above that preferably has a molecular weight of not less than 1 kDa but not more than 5,000 kDa in an amount of not less than 0.01 mg/mL but not more than 100 mg/mL, N-hydroxysuccinic acid imide [NHS] in an amount of not less than 0.01 mM but not more than 300 mM and water-soluble carbodiimide [WSC] in an amount of not less than 0.01 mM but not more than 500 mM for not shorter than 0.2 hours but not longer than 3. 0 hours, whereby the carboxymethyl dextran can be immobilized onto the SAM, and a "sensor chip substrate" is obtained.

The density of the resulting solid phase layer can be controlled by the number of reaction sites (number of functional groups of SAM), the ionic strength and pH of the reaction solution, and the WSC concentration to the number of carboxyl groups of the carboxymethyl dextran molecules. The mean film thickness of the solid phase layer can be controlled by the molecular weight of carboxymethyl dextran and the reaction time.

<Assay method>

The exemplary assay method comprises at least the following steps (a) to (d), and preferably further comprises a washing step.

Step (a) : a step of bringing a specimen into contact with the SPFS sensor chip according to the present invention.
Step (b): a step of further allowing a conjugate of a ligand which may be the same as or different from the ligand contained in the SPFS sensor chip and a fluorescent dye to react with the SPFS sensor chip obtained through the step (a).
Step (c) : a step of irradiating the SPFS sensor chip obtained through the step (b) with laser light from the other surface of the transparent support, on said surface the metal thin film not being formed, through a prism to measure the quantity of fluorescence emitted from the excited fluorescent dye.
Step (d): a step of calculating the quantity of an analyte contained in the specimen from the measurement result obtained in the step (c).

Washing step: a step of washing the surface of the SPFS sensor chip obtained through the step (a) and/or the surface of the SPFS sensor chip obtained through the step (b).

[Step (a)]

The step (a) is a step of bringing a specimen into contact with the SPFS sensor chip according to the present invention.

(Specimen)

Examples of the "specimens" include blood (serum, plasma), urine, snivel, saliva, stool and coelomic fluids (cerebrospinal fluid, peritoneal fluid, pleural fluid, etc.). The specimen may be used after it is properly diluted with a desired solvent, a buffer solution or the like. Of these specimens, blood, serum, plasma, urine, snivel and saliva are preferable.

(Contact)

A preferred embodiment of the "contact" is an embodiment in which the SPFS sensor chip and the specimen are brought into contact with each other in such a state that a specimen is contained in a transport liquid which circulates in a flow path and only one surface of the SPFS sensor chip, onto said surface the first ligand having been immobilized, is immersed in the transport liquid. It is enough just to bring the specimen into contact with the first ligand so that the analyte in the specimen may be captured by the first ligand, and the flow path does not necessarily have to be provided.
The "flow path" is polygonal tubular-like or cylindrical (tubular) as described above, and it is preferable that the flow path has a polygonal tubular-like structure in the vicinity of the place where the SPFS sensor chip is installed and has a cylindrical (tubular) structure in the vicinity of the place where a chemical liquid is sent.
As the material of the flow path, a homopolymer or copolymer containing methyl methacrylate, styrene or the like as a raw material, or polyolefin such as polyethylene is preferably used for the SPFS sensor chip zone or the flow path roof, and a polymer, such as silicone rubber, Teflon (registered trademark), polyethylene or polypropylene, is preferably used for the chemical liquid sending zone.
The flow path of the SPFS sensor chip zone preferably has a section having a length and a width each of which is about 100 nm to 1 mm, from the viewpoints that the contact efficiency with the specimen is raised and the diffusion distance is shortened in the SPFS sensor chip zone.
As a method to fix the SPFS sensor chip to the flow path in the case of small-scale lot (laboratory level), preferable is a method comprising first compression-bonding a polydimethylsiloxane [PDMS] sheet having a flow path height of 0.5 mm to the metal thin film surface of the SPFS sensor chip in such a manner that the metal thin film part of the SPFS sensor chip is surrounded by the sheet and then fixing the polydimethylsiloxane [PDMS] sheet and the SPFS sensor chip to each other with a fastener such as a screw.
For fixing the SPFS sensor chip to the flow path in the case of large-scale lot (factory level) industrially produced, a sensor substrate is formed in an integrally molded plastic product, or a sensor substrate prepared separately is fixed, then immobilization of SAM, a solid phase layer and a ligand onto the metal thin film surface is carried out (preferably, immobilization of a spacer layer composed of dielectric is also carried out), and thereafter, the flow path is capped with an integrally molded plastic product corresponding to the flow path roof. If necessary, a prism can be united with the flow path.

The "transport liquid" is preferably the same liquid as a solvent or a buffer solution for diluting the specimen, and examples thereof include a phosphoric acid-buffered physiological salt solution [PBS], a tris-buffered physiological salt solution [TBS] and an HEPES-buffered physiological salt solution [HBS], without limiting thereto.

The temperature of the transport liquid and the circulation time for circulating the transport liquid vary depending upon the type of the specimen, etc. and are not specifically restricted, but usually is 20 to 40°C × 1 to 60 minutes, and preferably is 37°C × 5 to 15 minutes.

The total quantity of the transport liquid, that is, the volume of the flow path, is usually 0.001 to 20 mL, preferably 0.1 to 1 mL.

The flow rate of the transport liquid is usually 1 to 2,000 μL/min, preferably 5 to 500 μL/min.

[Washing step]

The washing step is a step of washing the surface of the SPFS sensor chip obtained through the step (a) and/or the surface of the SPFS sensor chip obtained through the step (b).

As the wash liquid for use in the washing step, for example, a wash liquid which is obtained by dissolving a surface active agent such as Tween 20 or Triton X100 in the same solvent or buffer solution as used in the reactions of the step (a) and the step (b) and preferably contains 0.00001 to 1% by weight of the surface active agent or a wash liquid containing 10 to 500 mM of a salt such as sodium chloride or potassium chloride is desirable. A buffer solution of low pH, such as 10 mM Glycine HCl having pH of 1.5 to 4.0, may be used.

The temperature and the flow rate for circulating the wash liquid are preferably the same temperature and flow rate as used for circulating the transport liquid in the step (a).

The time for circulating the wash liquid is usually 0.5 to 180 minutes, preferably 5 to 60 minutes.

[Step (b)]

The step (b) is a step of further allowing a conjugate of a ligand (second ligand) which may be the same as or different from the ligand (first ligand) contained in the SPFS sensor chip and a fluorescent dye to react with the SPFS sensor chip obtained through the step (a), preferably obtained through the washing step.

(Fluorescent dye)

"Fluorescent dye" is a general name for substances which emit fluorescence when irradiated with predetermined excitation light or when excited utilizing electric field effect, and the "fluorescence" includes various luminescence such as phosphorescence.

The type of the fluorescent dye for use in the present invention is not specifically restricted, and any of publicly known fluorescent dyes may be used as far as it is not subject to quenching attributed to light absorption by the metal thin film. In general, preferable is a fluorescent dye enabling use of a spectrofluoro-photometer equipped with a filter rather than use of a monochromometer and having large stokes shift that enhances detection efficiency.

Examples of such fluorescent dyes include fluorescent dyes of fluorescein family (manufactured by Integrated DNA Technologies, Inc.), fluorescent dyes of polyhalofluorescein family (manufactured by Applied Biosystems Japan Ltd.), fluorescent dyes of hexachlorofluorescein family (manufactured by Applied Biosystems Japan Ltd.), florescent dyes of coumarin family (manufactured by Invitrogen Japan K.K.), fluorescent dyes of rhodamine family (manufactured by GE Healthcare Bioscience Co., Ltd.), fluorescent dyes of cyanine family, fluorescent dyes of indocarbocyanine family, fluorescent dyes of oxazine family, fluorescent dyes of thiazine family, fluorescent dyes of squaraine family, fluorescent dyes of chelated lanthanide family, fluorescent dyes of BODIPY (registered trademark) family (manufactured by Invitrogen Japan K.K.), fluorescent dyes of naphthalenesulfonic acid family, fluorescent dyes of pyrene family, fluorescent dyes of triphenylmethane family, and Alexa Fluor (registered trademark) dye series (manufactured by Invitrogen Japan K.K.). Further, fluorescent dyes described in U.S. Patent No. 6,406,297, U.S. Patent No. 6,221,604, U.S. Patent No. 5,994,063, U.S. Patent No. 5,808,044, U.S. Patent No. 5,880,287, U.S. Patent No. 5,556,959 and U.S. Patent No. 5, 135, 717 can be also used in the present invention.

Absorption wavelengths (nm) and emission wavelengths (nm) of typical fluorescent dyes included in these families are set forth in Table 1.

Table 1

| Fluorescent dye | Family | Absorption wavelength (nm) | Emission wavelength (nm) |
|---|---|---|---|
| Aminomethylcoumarin; AMCA | coumarin | 350 | 450 |

(continued)

| Fluorescent dye | Family | Absorption wavelength (nm) | Emission wavelength (nm) |
|---|---|---|---|
| Cy2 (registered trademark) | cyanine | 492 | 510 |
| Fluorescein Isothiocyanate; FITC | fluorescein | 492 | 520 |
| Cy3 (registered trademark) | indocarbocyanine | 550 | 570 |
| Tetramethylrhodamine Isothiocyanate; TRITC | rhodamine | 550 | 570 |
| Rhodamine Red-X; RRX | | 570 | 590 |
| Texas Red; TR | | 596 | 620 |
| Cy5 (registered trademark) | cyanine | 650 | 670 |
| Alexa Fluor (registered trademark) 647 | cyanine | 650 | 665 |

The fluorescent dyes are not limited to the above organic fluorescent dyes. For example, fluorescent dyes of rare earth complex systems such as Eu and Tb can also become fluorescent dyes for use in the present invention. The rare earth complexes have characteristics that they generally have a large difference between the excitation wavelength (about 310 to 340 nm) and the emission wavelength (Eu complex: near 615 nm, Tb complex: near 545 nm) and the fluorescence lifetime is as long as several hundreds micro seconds. An example of a commercially available fluorescent dye of the rare earth complex system is ATBTA-Eu$^{3+}$.

In the present invention, it is desirable to use a fluorescent dye having a wavelength of maximum fluorescence emission in the wavelength region in which the absorption by a metal contained in the metal thin film is low when the later-described measurement of fluorescence quantity is carried out. For example, when gold is used for the metal thin film, it is desirable to use a fluorescent dye having a wavelength of maximum fluorescence emission of not less than 600 nm in order to minimize the influence of absorption by the metal thin film. In this case, therefore, it is particularly desirable to use a fluorescent dye having a wavelength of maximum fluorescence emission in the near infrared region, such as Cy5 or Alexa Fluor (registered trademark) 647. Such a fluorescent dye having a wavelength of maximum fluorescence emission in the near infrared region is useful also in the case of using blood as a specimen, from the viewpoint that the influence of absorption by iron derived from a blood cell component in blood can be minimized. On the other hand, when silver is used for the metal thin film, it is desirable to use a fluorescent dye having a wavelength of maximum fluorescence emission of not less than 400 nm.

These fluorescent dyes may be used singly or in combination of two or more kinds.

(Conjugate of second ligand and fluorescent dye)

When a secondary antibody is used as a ligand, the "conjugate of a ligand (second ligand) which may be the same as or different from the ligand (first ligand) contained in the SPFS sensor chip according to the present invention and a fluorescent dye" is preferably an antibody capable of recognizing an analyte (target antigen) contained in the specimen and being bonded thereto.

In the exemplary assay method, the second ligand is a ligand that is used for the purpose of carrying out labeling of the analyte with the fluorescent dye, and may be the same as or different from the aforesaid first ligand. When the primary antibody used as the first ligand is a polyclonal antibody, the secondary antibody used as the second ligand may be a monoclonal antibody or a polyclonal antibody. However, when the primary antibody is a monoclonal antibody, the secondary antibody is desired to be a monoclonal antibody or a polyclonal antibody that recognizes an epitope that is not recognized by the primary antibody.

Also preferable is an example wherein a composite obtained by previous bonding of a second analyte (competitive antigen, this antigen is different from the target antigen), which competes with the analyte (target antigen) contained in the specimen, and the secondary antibody to each other is used. Such an example is preferable because the quantity of fluorescence signal and the quantity of the target antigen can be made proportional to each other.

In the case where a secondary antibody is used as the second ligand, examples of processes for preparing a conjugate of the second ligand and the fluorescent dye include a process comprising giving a carboxyl group to the fluorescent dye first, actively esterifying the carboxyl group by the use of water-soluble carbodiimide [WSC] (e.g., 1-ethyl-3-(3-dimethylaminopropyl)carbodiimide hydrochloride [EDC]) and N-hydroxysuccinic acid imide [NHS], then subjecting the actively esterified carboxyl group and the amino group of the secondary antibody to dehydration reaction by the use of water-soluble carbodiimide to perform immobilization; a process comprising allowing a secondary antibody having isothiocyanate and an amino group and the fluorescent dye to react with each other to perform immobilization; a process comprising allowing a secondary antibody having sulfonyl halide and an amino group and the fluorescent dye to react with each other to perform immobilization; a process comprising allowing a secondary antibody having iodoacetamide and a thiol group and the fluorescent dye to react with each other to perform immobilization; and a process comprising allowing a biotinylated fluorescent dye and a streptavidin-bonded secondary antibody (or streptoavidin-bonded fluorescent dye and biotinylated secondary antibody) to react with each other to perform immobilization.

The concentration of the thus prepared conjugate of the second ligand and the fluorescent dye in the transport liquid is preferably 0.001 to 10,000 μg/mL, more preferably 1 to 1,000 μg/mL.

The temperature, the time and the flow rate for circulating the transport liquid are the same as those in the step (a).

[Step (c)]

The step (c) is a step of irradiating the SPFS sensor chip obtained through the step (b) with laser light from the other surface of the transparent support, on said surface the metal thin film not being formed, (optionally through a prism) to measure the quantity of fluorescence emitted from the excited fluorescent dye.

(Optical system)

The light source for use in the exemplary assay method is not specifically restricted provided that it can cause plasmon excitation on the metal thin film, but it is preferable to use laser light as the light source from the viewpoints of singleness of wavelength distribution and intensity of optical energy. It is desirable to control energy and the photon quantity of the laser light through an optical filter immediately before the laser light enters the prism.

By the irradiation with laser light, surface plasmons are generated on the surface of the metal thin film under the conditions of attenuated total reflection [ATR]. By virtue of the field enhancement effect of the surface plasmons, the fluorescent dye is excited by the photons the quantity of which has been increased to several tens to several hundreds times the photon quantity for the irradiation. The increase of the photon quantity due to the field enhancement effect depends upon the refractive index of the transparent support, the metal species of the metal thin film and the film thickness thereof, but in the case of gold, the photon quantity is usually increased to about 10 to 20 times.

The fluorescent dye absorbs light to excite electrons in its molecules and becomes in the first excited electronic state in a short period of time, and when the fluorescent dye returns to the ground state from this state (level), fluorescence of wavelength corresponding to an energy difference between those states is emitted.

The "laser light" is, for example, LD of 0.001 to 1,000 mW having a wavelength of 200 to 900 nm or semiconductor laser of 0.01 to 100 mW having a wavelength of 230 to 800 nm (resonance wavelength is determined by the metal species used for the metal thin film).

The "prism" is used in order that the laser light having passed through various filters may efficiently enter the SPFS sensor chip, and preferably has the same refractive index as that of the transparent support. In exemplary embodiments of the present invention, various prisms capable of setting the total reflection conditions can be properly selected, so that there is no specific limitation on the angle and the shape of the prism used. For example, a 60° dispersion prism may be used. Examples of commercial products of such prisms include the same ones as the aforesaid commercial products of the "glass transparent supports".

The "optical filter" is, for example, a neutral density [ND] filter or a diaphragm lens. The "neutral density [ND] filter" (or ND filter) is used for the purpose of controlling the quantity of incident laser light. Particularly when a detector having a narrow dynamic range is used, the ND filter is preferably used in order to carry out high-accuracy measurement.

The "polarizing filter" is used in order to change the laser light to p-polarized light that efficiently generates surface plasmons.

The "cut filter" is a filter to remove optical noises, such as external light (illumination light outside the device), excitation light (transmitted light component of excitation light), stray light (scattered light component of excitation light in each place) and scattered light of plasmon (scattered light originating from excitation light and generated by the influence of a structure or a deposit on the SPFS sensor chip surface), and intrinsic fluorescence of the fluorescent dye. The cut filter is, for example, an interference filter or a color filter.

The "condenser lens" is used for the purpose of efficiently condensing fluorescence signals to a detector, and may be an arbitrary condenser system. As a simple condenser system, a commercially available objective lens (e.g., objective lens manufactured by Nikon Corporation or Olympus Corporation) that is used in a microscope or the like may be used for this purpose. The magnifying power of the objective lens is preferably 10X to 100X.

As the "SPFS detection part", a photomultiplier (Photomultiplier manufactured by Hamamatsu Photonics K.K.) is preferable from the viewpoint of super high sensitivity. A CCD image sensor capable of performing multi-point measurement is also preferable because the result can be seen as an image and optical noise can be easily removed, though its sensitivity is lowered as compared with the photomultiplier.

[Step (d)]

The step (d) is a step of calculating the quantity of an analyte contained in the specimen from the measurement result obtained in the step (c).

More specifically, the step (d) is a step in which a calibration curve is made by performing measurement using a target antigen or a target antibody of known concentration, and based on the calibration curve thus made, the quantity of an analyte (quantity of target antigen or target antibody) in the specimen to be measured is calculated from the measurement signal.

(Analyte)

The "analyte" is a molecule or a molecular fragment capable of being specifically recognized by (or recognizing) the first ligand and being bonded thereto. Examples of such "molecules" or "molecular fragments" include nucleic acids (DNA, RNA, polynucleotide, oligonucleotide, PNA (peptide nucleic acid), etc. which may be single-stranded or double-stranded, or nucleoside, nucleotide and their modified molecules), proteins (polypeptide, oligopeptide, etc.), amino acids (including modified amino acids), saccharides (oligosaccharide, polysaccharides, sugar chain, etc.), lipid, and modified molecules and complex of these substances, without limiting thereto. Specifically, the analyte maybe carcinoembryonic antigen such as AFP [α-fetoprotein), a tumor marker, a signal transmitting substance, hormone or the like, and is not specifically restricted.

(Amount of change of assay signal)

When the signal measured before the step (b) is taken as a "blank signal", the amount of change of an assay signal represented by the following formula can be calculated in the step (d).

Amount of change of signal = | (assay fluorescence signal) - (blank signal) |

<Assay device>

The exemplary assay device comprises at least the aforesaid SPFS sensor chip, and is used in the above exemplary assay method.

Such a device further comprises, for example, a light source of laser light, various optical filters, a prism, a cut filter, a condenser lens and a surface plasmon-field enhanced fluorescence [SPFS] detection part, in addition to the SPFS sensor chip, and when a specimen liquid, a wash liquid, a labeled antibody liquid or the like is dealt with, the device preferably has a liquid transport system combined with the SPFS sensor chip. The liquid transport system may be, for example, a micro-flow path device connected to a liquid transport pump.

The assay device may further comprises a surface plasmon resonance [SPR] detection part, namely, a photodiode as a light receiving sensor for SPR, an angle variation part for controlling optimum angles of SPR and SPFS (in order to determine the attenuated total reflection [ATR] conditions by a servomotor, photodiode and light source are synchronized with each other to make angle variation of 45 to 85° possible; resolution is preferably not less than 0.01°), a computer for processing information having been input into the SPFS detection part, etc.

Preferred examples of the light source, the optical filter, the cut filter, the condenser lens and the SPFS detection part are the same as those described above.

The "liquid transport pump" is, for example, a micro pump that is preferable in the case of a slight amount of a transport liquid, a syringe pump that has high transport accuracy and small pulsation but cannot perform circulation, a tube pump that is simple and has excellent handling property but sometimes has difficulty in liquid transport of a slight amount, or the like.

<Assay kit>

The exemplary assay kit comprises at least a sensor chip substrate which comprises a transparent support, a metal thin film formed on one surface of the transparent support, a self-assembled monolayer [SAM] formed on a surface of the metal thin film, said surface not being in contact with the transparent support, and a solid phase layer formed on a surface of the SAM and having a three-dimensional structure, said surface not being in contact with the thin film, and which is used in the aforesaid SPFS sensor chip. The assay kit preferably includes all the members necessary for performing the aforesaid assay method, except an analyte such as antigen, a specimen and a secondary antibody.

In the exemplary assay kit, a primary antibody may have been immobilized in advance in the solid phase layer of the SPFS sensor chip substrate.

By using the exemplary assay kit, a specimen, such as blood, plasma or serum, and an antibody against a specific tumor marker, the content of the specific tumor marker can be detected with high sensitivity and high accuracy. From this result, presence of non-invasive carcinoma (carcinoma in situ) in the preclinical stage, which cannot be detected by palpation or the like, can be foreseen with high accuracy.

Such an assay kit may include, for example, water-soluble carbodiimide [WSC] (e.g., 1-ethyl-3-(3-dimethylaminopropyl)carbodiimide hydrochloride [EDC]) and N-hydroxysuccinic acid imide [NHS] used for immobilizing an antibody in the solid phase layer, a fluorescent dye, a solvent or a diluent for dissolving or diluting a specimen, various reaction reagents used for allowing the SPFS sensor chip to react with a specimen, and wash liquids, in addition to the SPFS sensor chip substrate. The exemplary assay kit can also include various equipments and materials necessary for performing the exemplary assay method, and the above-mentioned "assay device".

The assay kit may further include, as kit elements, a set of necessary equipments and materials, such as a standard substance for making a calibration curve, description and a microtiter plate capable of performing simultaneous processing of plural specimens.

Examples

The present invention is described in more detail with reference to the following examples, but it should be construed that the present invention, defined by the appended claim, is in no way limited to those examples.

[Preparation Example 1] (Preparation of Alexa Fluor (registered trademark) 647-labeled secondary antibody)

As a secondary antibody, anti-$\alpha$-fetoprotein [AFP] monoclonal antibody (1D5; 2.5mg/mL, manufactured by Japan Clinical Laboratories, Inc.) was biotinylated using a commercially available biotinylation kit (manufactured by Dojindo Laboratories). The procedure was carried out in accordance with the protocol attached to the kit.

Next, a solution of the resulting biotinylated anti-AFP monoclonal antibody and a streptavidin-labeled Alexa Fluor (registered trademark) 647 (manufactured by Molecular Probes) solution were mixed, and the mixture was stirred and mixed for 60 minutes at 4°C to perform reaction.

Finally, the unreacted antibody and the unreacted reaction enzyme were purified by means of a molecular weight cut filter (manufactured by Nippon Millipore K.K.) to obtain an Alexa Fluor (registered trademark) 647-labeled anti-AFP monoclonal antibody solution. The resulting antibody solution was subjected to determination of protein concentration and then stored at 4°C.

[Preparation Example 2] (Preparation of substrate)

A glass transparent support ("S-LAL 10" manufactured by Ohara Inc.) having a refractive index [$n_d$] of 1.72 and a thickness of 1 mm was subjected to plasma cleaning, then a chromium thin film was formed on one surface of the support by sputtering, and thereafter, a gold thin film was formed on its surface by sputtering. The thickness of the chromium thin film was 1 to 3 nm, and the thickness of the gold thin film was 42 to 47 nm.

A half-width of the resulting substrate was measured as a half-width ($\beta$), and the value obtained was 7.6°.

[Example 1]

(Production of SPFS sensor chip (C))

The substrate obtained in Preparation Example 2 was immersed in 10 mL of an ethanol solution containing 10-amino-1-decanethiol having been adjusted to 1 mM, for 24 hours to form SAM on one surface of the gold thin film. This sensor substrate was taken out of the ethanol solution and washed with each of ethanol and isopropanol and then dried by the use of an air gun.

Subsequently, the sensor substrate on which SAM had been formed was immersed in an MES-buffered physiological salt solution [MES] (ionic strength: 10 mM) of pH 7.4 containing 1 mg/mL of carboxymethyl dextran [CMD] having a molecular weight of 500,000, 0.5 mM of N-hydroxysuccinic acid imide [NHS] and 1 mM of water-soluble carbodiimide [WSC] for 1 hour to immobilize the CMD onto the SAM, and the substrate was then immersed in a 1N NaOH aqueous solution for 30 minutes to hydrolyze the unreacted succinic acid ester.

Subsequently, the above substrate was immersed in MES containing 50 mM of NHS and 100 mM of WSC for 1 hour and then immersed in an anti-AFP monoclonal antibody (1D5, 2.5 $\mu$g/mL, manufactured by Japan Clinical Laboratories, Inc.) solution for 30 minutes to form a solid phase of the primary antibody onto CMD.

Further, using PBS containing 1% by weight of bovine serum albumin [BSA] and 1 M of aminoethanol, circulating liquid transport was carried out for 30 minutes to perform non-specific adsorption inhibition treatment. The half-width ($\alpha$) was 8.6°. The fluctuation ratio of a half-width was 13%. The fluctuation ratio was calculated using a half-width of the substrate obtained in preparation Example 2 as the half-width ($\beta$).

(Performance of assay method)

Using the SPFS sensor chip (C) produced as above, the following assay method was performed.

First, on the SPFS sensor chip having the solid-phase antibody, a polydimethylsiloxane [PDMS] sheet having a flow path height of 0.5 mm and having a hole of proper shape and size was provided, and around this PDMS sheet, a silicone rubber spacer (this silicone rubber spacer did not come into contact with a transport liquid) was arranged. On the PDMS sheet and the silicone rubber spacer, a PMMA substrate having a hole for transport liquid introduction and a hole for transport liquid discharge previously formed was arranged in such a manner that these holes were positioned inside the region surrounded by the PDMS sheet (at this time, the PMMA substrate was arranged so that the surface of the solid-phase antibody might be inside the flow path). These members were compression-bonded to each other outside the flow path, and the PMMA substrate, the flow path sheet (i.e., the above-mentioned PDMS sheet) and the SPFS sensor chip (C) were fixed together with a screw.

In the step (a), 0.1 mL of a PBS solution containing 0.1 ng/mL of AFP as a target antigen was circulated to the SPFS sensor chip obtained as above for 25 minutes.

In the step (b), a tris-buffered physiological salt solution [TBS] containing 0.05% by weight of Tween 20 was circulated as a transport liquid for 10 minutes to perform washing, and thereafter, 0.1 mL of the Alex Fluor (registered trademark) 647-labeled secondary antibody (PBS solution having been adjusted so as to have a concentration of 2 $\mu$g/mL) obtained in Preparation Example 1 was circulated for 5 minutes.

In the step (c), TBS containing 0.05% by weight of Tween 20 was first circulated as a transport liquid for 10 minutes to perform washing. The plasmon excitation sensor was irradiated with laser light (640 nm, 40 $\mu$W) through a prism (manufactured by Sigma Koki Co., Ltd.) from the other surface of the glass transparent support, on said surface the metal thin film not being formed, and the quantity of fluorescence emitted from the excited fluorescent dye was detected by a photomultiplier [PMT] to measure the quantity of light (signal value). The resulting signal value was taken as an "assay signal".

The result obtained is set forth in Table 2

In the step (d), the quantity of the analyte contained in the specimen was calculated from the result of the fluorescence quantity measurement obtained in the step (c).

[Example 2]

An SPFS sensor chip (D) was produced in the same manner as in Example 1, except that CMD having a molecular weight of 150,000 was used instead of CMD having a molecular weight of 500,000. This SPFS sensor chip (D) had a half-width ($\alpha$) of 7.9°, and therefore, the fluctuation ratio of a half-width was 4%. Using the SPFS sensor chip (D), an assay method was performed in the same manner as in Example 1. The result obtained is set forth in Table 2.

[Example 3]

An SPFS sensor chip (E) was produced in the same manner as in Example 1, except that the concentration (1 mg/mL) of CMD having a molecular weight of 500, 000 was changed to 10 mg/mL, the concentration (0.5 mM) of NHS was changed to 100 mM, the concentration (1 mM) of WSC was changed to 100 mM, and pH 7.4 of MES (ionic strength: 10 mM) was changed to pH 6.0 (ionic strength: 10 mM) . This SPFS sensor chip (E) had a half-width ($\alpha$) was 8.8°, and therefore, the fluctuation ratio of a half-width was 29%. Using the SPFS sensor chip (E), an assay method was performed in the same manner as in Example 1. The result obtained is set forth in Table 2.

[Comparative Example 1]

(Production of SPFS sensor chip (A))

An SPFS sensor chip (A) was produced in the same manner as in Example 1, except that in the formation of SAM, 10-carboxy-1-decanethiol was used instead of 10-amino-1-decanethiol, and the antibody was directly immobilized onto SAM without forming a solid phase layer using CMD. The half-width ($\alpha$) was 7.7°, and therefore, the fluctuation ratio of a half-width was 1.3%.

The half-width obtained by the use of the SPFS sensor chip thus produced cannot be strictly said to be a half-width ($\alpha$), but the half-width obtained is taken as a half-width ($\alpha$) in Table 2 for convenience.

(Performance of assay method)

An assay method was performed in the same manner as in Example 1, except that the SPFS sensor chip (A) produced as above was used instead of the SPFS sensor chip (C) produced in Example 1. The result obtained is set forth in Table 2.

[Comparative Example 2]

(Production of SPFS sensor chip (B))

An SPFS sensor chip (B) was produced in the same manner as in Example 1, except that 100 mg/mL of CMD was used instead of 1 mg/mL of CMD, 100 mM of NHS was used instead of 0.5 mM of NHS, 100 mM of WSC was used instead of 1 mM of WSC, and pH 7.4 of MES (ionic strength: 10 mM) was changed to pH 6. 0 (ionic strength: 150 mM) . The half-width ($\alpha$) was 13.6°, and therefore, the fluctuation ratio of a half-width was 79%.

(Performance of assay method)

An assay method was performed in the same manner as in Example 1, except that the SPFS sensor chip (B) produced as above was used instead of the SPFS sensor chip (C) produced in Example 1. The result obtained is set forth in Table 2.

Table 2: Properties of SPFS sensor chip obtained and results obtained by performing assay method

| | | Ex. 1 | Ex. 2 | Ex. 3 | Comp. Ex. 1 | Comp. Ex. 2 |
|---|---|---|---|---|---|---|
| SPFS sensor chip | | (C) | (D) | (E) | (A) | (B) |
| Half-width ($\alpha$) | | 8.6° | 7.9° | 8.8° | 7.7° | 13.6° |
| Fluctuation ratio represented by {half-width ($\alpha$) - ($\beta$)} / ($\beta$)$\times$100 | | 13% | 4% | 29% | 1.3% | 79% |
| Solid phase layer or SAM only | Film thickness in swelling (measured by atomic force microscope [AFM]) | <70nm | <40nm | <60nm | <10nm | <20nm |
| | Density of CMD [ng/mm$^2$] | 1.9 | 1.7 | 2.3 | - | 1.7 |
| Ligand (anti-AFP antibody) | Solid-phase antibody density [mol/cm$^2$] | several pico | several pico | several pico | several pico | several tens pico |
| | Effective antibody density [mol/cm$^2$] | 1 pico | 1 pico | 1 pico | several hundred s femto | several hundred s femto |
| | Effective antibody density ratio | 50% | 50% | 50% | 10-20% | 20-30% |
| Assay signal | Photon quantity [/sec] | $4.9\times10^4$ -$5.7\times10^6$ | $4.3\times10^4$ -$4.7\times10^6$ | $3.9\times10^6$ -$4.5\times10^6$ | $2.2\times10^6$ -$2.5\times10^6$ | $3,0\times10^5$ -$3.0\times10^6$ |
| | Coefficient of variation [CV] (= standard deviation [SD] /average value [Ave]$\times$100) | 15% (N=12) | 12% (N=9) | 14% (N=15) | 10% (N=6) | 119% (N=6) |

[Consideration]

It can be seen from Table 2 that the assay signals obtained in Example 1 (fluctuation ratio: 13%), Example 2 (fluctuation ratio: 4%) and Example 3 (fluctuation ratio: 29%) were significantly larger than those obtained in Comparative Example 1 (fluctuation ratio: 1.3%) and Comparative Example 2 (fluctuation ratio: 79%), and the coefficients of variation [CV], i.e., variability, of Examples 1 to 3 were significantly lower than CV of Comparative Example 2 and were almost equivalent to CV of Comparative Example 1.

That is to say, in the case of the SPFS sensor chips of Examples 1 to 3, the assay signals were free from non-uniformity (that is, the signals were stable) and were remarkably increased, in spite that the SPFS sensor chips used a dextran layer similarly to Comparative Example 2. Therefore, by the use of the SPFS sensor chips of the present invention, improvement in detection stability and high-sensitive detection can be achieved.

Industrial Applicability

The exemplary assay method using the SPFS sensor chip according to the present invention, defined by the appended claim, is a method capable of performing detection with high sensitivity and high accuracy, so that, for example, even an extremely slight amount of a tumor marker contained in blood can be detected, and from this result, presence of non-invasive carcinoma (carcinoma in situ) in the preclinical stage, which cannot be detected by palpation or the like, can be foreseen with high accuracy.

Reference Signs List

1: transparent support
1': transparent plate
2: metal thin film
2': metal film
3: SAM
4: carboxymethyl dextran [CMD]
4': dextran layer
5: ligand (antibody)
6: analyte (antigen)
7: fluorescent-labeled antibody
8: fluorescent dye
9: solid phase layer
11: derivative block
12: metal film
13: hydrophobic polymer compound whose surface has been coated with hydrogel
10: SPFS sensor chip
100: sensor unit
200: measuring chip

## Claims

1. A surface plasmon-field enhanced fluorescence spectroscopy [SPFS] sensor chip (10) comprising:

   a transparent support (1),
   a metal thin film (2) formed on one surface of the transparent support (1),
   a self-assembled monolayer [SAM] (3) formed on a surface of the metal thin film (2), said surface not being in contact with the transparent support (1) a solid phase layer (9) formed on a surface of the SAM (3) and having a three-dimensional structure, said surface not being in contact with the metal thin film (2), wherein the solid phase layer (9) contains a hydrophilic polymer, and
   a ligand (5) immobilized in the solid phase layer (9),
   **characterized in that** the SPFS sensor chip (10) has a fluctuation ratio, represented by the following formula, in the range from 0 to 30 %,

$$\{\text{half-width } (\alpha) - \text{half-width } (\beta)\}/\text{half-width } (\beta) \times 100$$

wherein

(i) the solid phase layer (9) has a density of less than 2 ng/mm$^2$;

(ii) the solid phase layer (9) has a mean film thickness of 3 to 80 nm;

(iii) the density of the ligand (5) immobilized in the solid phase layer (9) is in the range from 10 femto-mol/cm$^2$ to 100 pico-mol/cm$^2$;

(iv) the half-width ($\alpha$) is a half-width obtained from a graph on which a quantity of reflected light of light entering one surface of the transparent support (1) at a prescribed angle, the surface not being in contact with the metal thin film (2), as measured by a light quantity detector placed on the other surface side of the transparent support (1), is plotted against the angle, and the half-width ($\beta$) is a half-width of a substrate that is the SPFS sensor chip (10) including the transparent support (1) and the metal thin film (2) formed on one surface of the transparent support (1) but not including the SAM (3), the solid phase layer (9) and the ligand (5).

**Patentansprüche**

1. Ein Oberflächenplasmonenfeld-verstärkte Fluoreszenzspektroskopie [SPFS]-Sensor-Chip, der umfasst:

Einen transparenten Träger (1),
einen auf einer Oberfläche des transparenten Trägers (1) gebildeten Metalldünnfilm,
eine auf der Oberfläche des Metalldünnfilms (2) gebildete selbstorganisierte Monoschicht [SAM] (3), besagte Oberfläche nicht in Kontakt mit dem transparenten Träger (1) stehend,
eine auf einer Oberfläche der SAM (3) gebildete und über eine dreidimensionale Struktur verfügende Festphasenschicht (9), besagte Oberfläche nicht in Kontakt mit dem Metalldünnfilm (2) stehend, wobei die Festphasenschicht (9) ein hydrophiles Polymer und einen in der Festphasenschicht (9) immobilisierten Liganden (5) enthält, der dadurch charakterisiert ist, dass der SPFS-Sensor-Chip (10) ein durch die folgende Formel wiedergegebenes Fluktuationsverhältnis in dem Bereich von 0 bis 30 % hat,

$$\{Halbbreite\ (\alpha) - Halbbreite\ (\beta)\}/Halbbreite\ (\beta) \times 100$$

wobei

(i) die Festphasenschicht (9) eine Dichte von weniger als 2 ng/mm$^2$ hat;

(ii) die Festphasenschicht (9) eine mittlere Filmdichte von 3 bis 80 nm hat;

(iii) die Dichte des in der Festphasenschicht (9) immobilisierten Liganden (5) im Bereich von 10 Femto-mol/cm$^2$ bis 100 Pico-mol/cm$^2$ beträgt;

(iv) die Halbbreite ($\alpha$) die Halbbreite ist, die aus einem Graph erhalten wird, bei dem die Menge reflektierten Lichts von Licht, das in eine Oberfläche des transparenten Trägers unter einem vorgeschriebenen Winkel eintritt, die Oberfläche nicht in Kontakt mit dem Metalldünnfilm (2) stehend, wie gemessen durch einen auf die andere Oberflächenseite des transparenten Trägers (1) platzierten Lichtmengendetektor, gegen den Winkel aufgetragen wird,
und die Halbbreite ($\beta$) die Halbbreite eines Substrates ist, bei dem es sich um den SPFS-Sensor-Chip (10) handelt, der den transparenten Träger (1) und den auf einer Oberfläche des transparenten Trägers (1) gebildeten Metalldünnfilm (2), jedoch nicht die SAM (3), die Festphasenschicht (9) und den Liganden (5) beinhaltet.

**Revendications**

1. Puce de détection de spectroscopie par fluorescence améliorée par champ de plasmons de surface [SPFS] (10) comprenant :

un support transparent (1),
un film mince métallique (2) formé sur une surface du support transparent (1),
une monocouche auto-assemblée [SAM] (3) formée sur une surface du film mince métallique (2), ladite surface n'étant pas en contact avec le support transparent (1)
une couche en phase solide (9) formée sur une surface de la SAM (3) et ayant une structure tridimensionnelle, ladite surface n'étant pas en contact avec le film mince métallique (2), où la couche en phase solide (9) contient un polymère hydrophile,
et
un ligand (5) immobilisé dans la couche en phase solide (9),
**caractérisée en ce que**
la puce de détection SPFS (10) a un rapport de fluctuation représenté par la formule suivante dans la plage allant de 0 à 30%,

$$\{Demi\text{-}largeur\ (\alpha) - demi\text{-}largeur\ (\beta)\}/demi\text{-}largeur\ (\beta) \times 100$$

où

(i) la couche en phase solide (9) a une densité inférieure à 2 ng/mm$^2$ ;
(ii) la couche en phase solide (9) a une épaisseur de film moyenne allant de 3 à 80 nm ;
(iii) la densité du ligand (5) immobilisé dans la couche en phase solide (9) est dans la plage allant de 10 femtomoles/cm$^2$ à 100 picomoleslcm$^2$ ;

la demi-largeur ($\alpha$) est une demi-largeur obtenue à partir d'un graphique sur lequel une quantité de lumière réfléchie d'une lumière entrant dans une surface du support transparent (1) à un angle prescrit, la surface n'étant pas en contact avec le film mince métallique (2), telle que mesurée par un détecteur de quantité de lumière placé sur l'autre côté de surface du support transparent (1), est tracée par rapport à l'angle, et la demi-largeur ($\beta$) est une demi-largeur d'un substrat qui est la puce de détection SPFS (10) comportant le support transparent (1) et le film mince métallique (2) formé sur une surface du support transparent (1) mais ne comportant pas la SAM (3), la couche en phase solide (9) et le ligand (5).

[Fig.1]

[Fig.2]

Enhanced field

Immune reaction

SPFS signal

Antibody density,
Density of
solid phase layer

Optimum antibody density

[Fig.3]

(A)  (B)  (C)

1

2

5

4

3

[Fig.4]

[Fig.5]

[Fig.6]

[Fig.7]

[Fig.8]

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20080187462 A1
- JP 3294605 A
- WO 9005295 A
- JP 4292043 B
- JP 2005987770 A
- US 6406297 B
- US 6221604 B
- US 5994063 A
- US 5808044 A
- US 5880287 A
- US 5556959 A
- US 5135717 A